Europäisches Patentamt

(19) European Patent Office                    (11) Publication number:        **0 237 542**
Office européen des brevets                                             **B1**

(12)                        **EUROPEAN PATENT SPECIFICATION**

(45) Date of publication of patent specification: **30.01.91**    (51) Int. Cl.⁵: **A 61 K 9/50,** A 23 P 1/04,
                                                                   A 23 L 1/22
(21) Application number: **86905371.0**

(22) Date of filing: **17.09.86**

(86) International application number:
**PCT/GB86/00550**

(87) International publication number:
**WO 87/01587 26.03.87 Gazette 87/07**


(54) MICROCAPSULES.


| | |
|---|---|
| (30) Priority: **17.09.85 GB 8522963** | (73) Proprietor: **BIOCOMPATIBLES LTD.**<br>**2 Gray's Inn Square**<br>**London WC1R 5AF (GB)** |
| (43) Date of publication of application:<br>**23.09.87 Bulletin 87/39** | |
| | (72) Inventor: **HAYWARD, James, Arthur**<br>**4 Chuck Court Port Jefferson**<br>**New York, NY 11777 (US)**<br>Inventor: **LEVINE, Daniel, M.** |
| (45) Publication of the grant of the patent:<br>**30.01.91 Bulletin 91/05** | **28 Evergreen Road**<br>**Rocky Point, NY 11778 (US)**<br>Inventor: **SIMON, Sanford, R.**<br>**8 Oak Court** |
| (84) Designated Contracting States:<br>**AT BE CH DE FR GB IT LI LU NL SE** | **Selden, NY 11784 (US)** |
| (56) References cited:<br>EP-A-0 133 391<br>EP-A-0 140 085<br>EP-A-0 160 266<br>EP-A-0 162 724<br>WO-A-85/03640<br>WO-A-86/01404<br>DE-A-2 640 707 | (74) Representative: **Cresswell, Thomas Anthony**<br>**et al**<br>**J.A. Kemp & Co. 14 South Square Gray's Inn**<br>**London WC1R 5EU (GB)** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a new type of microcapsules for use as an aquacultural feedstuff.

The controlled delivery of substances from microscopic depots has been the object of a wide spectrum of industrial and biomedical research. A great variety of pharmacological dosage forms have been developed (reviewed by Rogers, 1982) to achieve prolonged or controlled action of incorporated or encapsulated drugs.

Liposomes have been heralded as an exciting solution to various drug delivery problems. They are lipid-water systems in which an aqueous volume is enclosed by a bilayer of amphipathic lipids. Lipid bilayers are themselves well structured two-dimensional solutions, consequently, dissolution of liposomes and loss of their payload occurs when liposomes are exposed to hydrodynamic shear, lipolytic enzymes and emulsifiers. Hydrophilic solutes may be entrapped within their enclosed volume, but their capacity to transport hydrophobic solutes is limited. Among the further defects of liposomes which have considerably limited their application as controlled release vehicles are their instability, variable leakage rates and propensity toward fusion, aggregation and precipitation. Despite enormous research efforts over the last 20 years, these limitations have severely restricted the practical application of liposomes for controlled release.

Alternative, non-liposomal microcapsules and microparticles (including "nano-particles" (Kreuter, 1983)) have been prepared from a variety of macromolecular colloids in which the active principles are dissolved, entrapped, encapsulated and/or to which the active principle is adsorbed or attached. Restrictions in the applications of microcapsules derive from toxicity of the monomer and/or polymer, contamination of the polymer by monomer, free-radical initiators and catalysts, extraction and/or denaturation of the payload by an organic phase, chemical modification of the payload during polymerisation, and variations in biodegradation.

It has long been the goal of aquaculturists to develop a micro-encapsulated diet. When aquatic animals are cultured in closed systems, there is a delicate balance between proper feeding and over-feeding. In the latter instance, excess food leads to bacterial fouling of the culture water which, in turn, leads to loss of the animals. Microencapsulation techniques permit diet delivery and control without the problem of fouling. Many types of materials are available for the preparation of microcapsules and microparticles.

The first use of microencapsulation techniques as an approach to producing artificial diets was 1974 when Jones et al. modified the original technique of Chang et al. (1966) for producing nylon-protein microcapsules. Nylon-protein microcapsules have been fed to a variety of organisms, including: larvae of the brine shrimp, Artemia (Jones et al. 1974; Jones et al. 1975; Jones and Gabbott, 1976); the shrimps, Macrobrachium rosenbergii, Crangon crangon, Palaemon elegans and palaemons merguiensis and the hermit crab, pagurus bernhardus (Jones et al. 1975); the shrimp, Penaeus japonicus (Jones et al. 1979a, 1979b); larvae of the oyster, Crassostrea virginica (Chu et al. 1982); the oyster, Crassostrea gigas (Gabbott et al. 1976; Langdon, 1977) and juveniles of the blue mussel, Mytilus edulis (Gabbott et al., 1976).

These results clearly demonstrate that nylon-protein microcapsules may be utilised for dietary supplementation in aquacultural systems. However, there are major disadvantages with this type of microcapsule. Only macromolecular dietary components can be encapsulated and retained in the capsule due to the high permeability of the capsule wall (Jones and Gabbott, 1976; Jones et al. 1976), and large amounts of lipid and hydrophobic components are lost from the aqueous diet phase to the organic solvent during the encapsulation process (Jones and Gabbott, 1976; Jones et al. 1979a).

Langdon and Waldock (1981) used gelatin-ethyl cellulose microcapsules to encapsulate carbohydrate in their studies of bivalve nutrition. Although these capsules are well suited for specific applications, their utility is limited. Ethyl cellulose microcapsules can be used only for encapsulating carbohydrate and protein and can not be used to encapsulate lipid because the technique requires the use of organic solvents, which remove the hydrophobic components from the microcapsules.

Gelatins are commonly used as binders in aquatic animal feeds (New, 1976), either singly or in mixtures. The bound material is crushed into suitably sized pieces for use. The pieces break up quickly in aqueous environments and tend to foul the water in a matter of hours. The use of gelatins for preparing microencapsulated or microparticulate diets for aquacultural purposes, however, began as recently as 1981 when Langdon and Waldock first used gelatin-acatia to encapsulate neutral oils.

The utility of gelatin-acatia as a matrix for diet delivery is restricted because only non-aqueous nutrients (e.g. lipids) may be entrapped. The gel forms semi-solid particles of a porous matrix which can not be used for the encapsulation of a total diet containing protein, carbohydrate and lipid because the aqueous elements are partially lost during preparation.

Calcium alginate gels have been used widely in the chemical and food industries as thickeners and to control texture. Published studies (Levine and Sulkin, 1984a,b) have demonstrated the versatility of calcium alginate microcapsules for encapsulating dietary particles and/or oils. Microcapsules of calcium alginate may be enriched with specific polyunsaturated fatty acids. The entrapped fatty acids remain tightly associated with the capsules and do not desorb nor escape at an appreciable rate. The calcium alginate system can be used to encapsulate powder and lipid simultaneously provided the powder is emulsified in the lipid.

Levine and co-workers have used calcium alginate microcapsules to study the nutritional requirements of brachyuran crab larvae (Levine, 1983, Levine et al. 1983, Levine and Sulkin, 1984a,b). "Empty" microcapsules are not nutritious in and of themselves. Survival and development rates of crab larvae were improved when a diet of live rotifers, Brachionus plicatilis, was supplemented with calcium-alginate microcapsules containing dietary additions. When the rotifer diet was fed in combination with encapsulated unsaturated fatty acids, ingestion of microcapsules resulted in assimilation of single microencapsulated long chain polyunsaturated fatty acids into megalopa (postlarval stage) total lipid.

These experiments collectively demonstrate that encapsulation of specific dietary components in calcium alginate microcapsules results in assimilation by crab larvae. Due to their high permeability, however, these microcapsules can not be used to encapsulate water-soluble components in solution (e.g. vitamins, amino acids, enzymes etc.).

All of the above mentioned gelling-hydrocolloid techniques share a common disadvantage, namely limited ability to produce a gelatin microcapsule that will retain water-soluble nutrients. Furthermore, because each type of microcapsule, with the exception of the calcium alginate system developed by Levine and co-workers, is specialised for encapsulating a specific dietary component (e.g. lipid or carbohydrate), no single method can be used to produce a complete artificial diet.

Langdon and co-workers (Langdon, 1983; Langdon and Siegfried, 1984; Langdon and Bolton, 1984) and Chu et al. (1983) have developed "lipid-walled" capsules specifically for encapsulating water-soluble nutrients. These capsules are composed of neutral lipids and must be fed with other types of microcapsules since they alone can not be used to deliver a complete diet. Langdon and co-workers have also developed a "microgel particle" that is similar to the calcium alginate microcapsule described by Levine (1983) except that they are produced by a solvent evaporation process rather than by spraying (Langdon and Levine, 1983; Langdon et al., 1985). These particles include a phospholipid mixture (soy lecithin) as a dietary supplement.

Wheatley et al. (1985) described a drug delivery system wherein prolonged release of myoglobin is achieved using a liposomal formulation of myoglobin entrapped in a matrix of alginate cross-linked by calcium ions.

It has now been discovered that nutritional components can be delivered by encapsulation in liposomes comprising nutritional lipids, the liposomes being protected by microencapsulation in a hydrocolloid matrix and that certain hydrocolloid matrices containing alginate and gelatin offer particular advantages in ease of encapsulation and control in release of nutritional payloads from entrapped liposomes.

Accordingly the present invention provides microcapsules for use as an aquacultural feedstuff each microcapsule comprising a payload entrapped in liposomes, the liposomes being encapsulated in a cured hydrocolloid matrix, wherein the payload comprises a nutritional component and the liposomes comprise at least one nutritional lipid.

Preferably the microcapsule matrix comprises alginate and gelatin.

Hereafter the term "lipogel microcapsule" will be used to denote a microcapsule according to the invention.

Permeation of the payload through the wall of the microcapsule may be regulated by the ratio of alginate to gelatin, their absolute concentrations in the microcapsule, the extent of cross-linking of the microcapsular matrix, and the chemistry of cross-linking.

Suitably the microcapsules have a hydrocolloid matrix or wall that is digestible and that permits the delivery of a complete diet. Types of colloids employed for generation of microcapsules include the gelling hydrocolloids such as agar, alginate, carrageenan, carboxymethyl cellulose, furcellaran, gelatin, pectin and xanthan, suitable polymerised organics, and the products formed by chemical cross-linking of natural materials such as human and bovine albumin, casein and gelatin. Combinations of different colloidal types, such as the acrylic dextrans, yield carriers which exhibit characteristics of each of the components.

Suitably the matrix comprises an alginate salt and gelatin in a ratio of from 99:1 to 1:99 by weight, preferably from 10:1 to 1:1 and most preferably about 3:1 by weight. The ratio of alginate to gelatin may be modified to modulate the permeability barrier posed by the matrix. Various soluble and insoluble salts of alginate may be employed; preferred soluble salts include sodium alginate and preferred insoluble salts include calcium alginate.

Without wishing to be bound by any theory it is believed that the gelatin content of the microcapsules may serve to modify the permeability of the capsular wall. In addition, the simultaneous presence of gelatin and alginate permits the formation of alginate-alginate, alginate-protein and protein-protein cross-links through the use of chemical cross-linkers such as formaldehyde.

The payload may be any nutritional component or a mixture of such components such as any hydrophilic or hydrophobic solute (excluding those which would solubilize the lipid bilayer) which can be entrapped in liposomes. The liposomes are formed of at least one nutritional lipid and may be composed of a mixture of lipids selected to entrap the payload and provide desired release characteristics.

The term "nutritional component" encompasses all materials which are nutritionally valuable as a constituent part of an aquatic organism's diet, whether as an essential component of that diet or otherwise. Thus examples of nutritional components are proteins, minerals, carbohydrates, vitamins and amino acids.

The liposomes are formed from at least one lipid which is a nutritional component, examples of which are the unsaturated fatty acids required as part of the diet fed to aquatic organisms in aquaculture. The composition of the entrapped liposomes may be controlled to achieve controlled release of the payload as a function of temperature, pH, or ion concentration. The liposome may be composed of any bilayer- or micelle-forming lipid. All lipids in these catagories may be suitable, regardless of their combined or individual permeability, since they may be combined to achieve the desired payload-release kinetics.

Liposomes may be prepared from essentially any class of phospholipid; variability in phospholipid class derives from each of the hydrophobic and hydrophilic domains of these amphipathic molecules. Individual phospholipids may be chosen based upon their permeability and release characteristics. Permeability of individual liposome preparations is dependent upon: extent of unsaturation, presence or absence of sterols, the temperature of their gel-liquid-crystalline phase transition, the ionic- and pH-dependance of their lamellar-hexagonal transition, the temperature-dependence of non-bilayer configurations, and the extent of monomer conversion of polymerizable phospholipids (Hayward et al., 1985).

The liposomes may be uni- or multilamellar, and they may be of any suitable size appropriate to the intended use of the lipogel microcapsules and the materials used to form the liposomes. Suitable lipid compositions for the liposomes include mixtures of lecithin (e.g. soy lecithin) or dipalmitoylphosphatidyl choline (DPPC) and cholesterol in a molar ratio of from 200:1 to 1:2, for instance 2:1 to 1:2, preferably about 1.1.

Polyunsaturated fatty acids and sterols must be supplied in aquacultural diets because of the inability of larvae to synthesise these compounds de novo. Normally, these exogenous lipids are supplied in multicomponent diets. Provision of the appropriate unsaturated lipids in a single-capsule diet may be attained using a phospholipid fraction of fish oil such as dogfish oil or menhaden oil, obtained from commercial fish processing plants and isolated by column chromatography (Bartlett, 1959), that is enriched in polyunsaturated fatty acid. Permeability of liposomes derived from polyunsaturated menhaden phospholipids may be regulated by the addition of increasing mole fractions of cholesterol, itself an obligatory component of the aquacultural diet.

Oxidation of the unsaturated lipids may be inhibited by the inclusion of commercially employed antioxidants such as alpha-tacopherol (vitamin E), butylated hydroxy toluene (BHT), ascorbic acid (vitamin C), nordihydroguaiaretic acid and others. The specified antioxidant may be chosen to enhance the nutritional value of the diet.

In addition to composition, the configuration of liposomal preparations may be varied to suit specific applications. A great variety of liposomal preparatory methods are available which differ in: ease of production, solvent and equipment requirements, and tolerance for different lipid classes. The liposomes produced by these different methodologies vary in: number of lamellae, aqueous volume enclosed per mole of phospholipid, resistance to shear, entrapment efficiency, diameter, and resistance to fusion/aggregation/precipitation (reviewed by Gregoriadis, 1984). The choice of liposomal configuration will be dictated largely by the intended application and the required profile of payload release.

Because of the unique encapsulation system of a cured matrix encapsulating liposomes entrapping nutritional components it is possible to produce a complete diet for an organism in a single formulation. Alternatively the lipogel microcapsules may be made up into the desired aquacultural diet by admixture with an appropriate carrier and optional accessory ingredients. The present invention therefore also provides a formulation comprising lipogel microcapsules and a carrier or diluent therefor. The invention also provides a process for producing formulations described above comprising bringing into association lipogel microcapsules and a carrier or diluent therefor. Preferably the payload and liposome constitute a complete diet for an organism such as a mollusc, crustacean, fish or mammal.

Lipogel microcapsules may be formed in any size appropriate to their intended use. Aquacultural microcapsules would typically have a microscopic particle size. A large number of liposomes will be encapsulated by the hydrocolloid matrix of each lipogel microcapsule.

The present invention also provides a process for producing lipogel microcapsules which process comprises

a) entrapping the payload in liposomes,

b) encapsulating the liposomes in a hydrocolloid matrix.

and

c) curing the matrix.

In step a) the liposomes may be formed by conventional processes such as those described by Gregoriadis (1984).

Step b) is accomplished by admixing the liposomes with an aqueous solution of the hydrocolloid matrix material.

In step c), curing or polymerization of the microcapsular matrix may be accomplished by chemical means (as in the calcium ion mediated precipitation of sodium alginate) or by physical means (as in the thermal gelling of the carrageenans). The desired particle size may be achieved by curing an atomised dispersion of the monomeric hydrocolloid, by extrusion methods, or by curing in bulk followed by crushing.

In a preferred embodiment of this process the payload is entrapped in the liposomes by reverse phase evaporation and the liposomes are then separated from unentrapped material and washed. The liposomes are then admixed with the hydrocolloid matrix material in aqueous solution and sprayed into a curing bath. Conveniently liposomes comprising a phospholipid are aerosolised or nebulised using conventional nebulising or aerosol-forming equipment and a suitable carrier gas such as compressed air. In this way droplets of matrix solution each containing a number of liposomes are formed and the matrix is then cured in the curing bath. For lipogel microcapsules having an alginate/gelatin matrix the curing bath preferably contains calcium chloride solution, for instance at a concentration in the range of from about 1% to about 20% w/v and the lipogel microcapsules reside in the curing bath for about 5 minutes in order to harden the matrix. The viscosity and texture of the lipogel microcapsules is dependent upon the extent (e.g., duration) of curing, the concentration and type of curing agent, the concentration of alginate, and the ratio of gelatin to alginate. After formation and curing, the lipogel microcapsules are separated from the curing bath and washed prior to storage or use. The lipogel microcapsules may be stored as a freeze-dried powder wherein the structural integrity of the microcapsule is maintained by the presence of high levels of carbohydrate.

The invention also provides a method for feeding aquatic livestock wherein the livestock are provided with lipogel microcapsules or a formulation thereof in partial or complete fulfilment of the dietary requirement of the livestock.

The invention will now be illustrated by the following Examples which are not intended to limit the invention in any way.

### EXAMPLE 1
#### (a) Production of Liposomes

Liposomes were constructed using cholesterol and (a) soy lecithin or (b) dipalmitoylphosphatidylcholine (DPPC)(1:1, mole:mole). The lipid was initially dissolved in chloroform (5 ml) in a 50 ml conical flask and dried to a thin film by evaporation under nitrogen. A solution of 6-carboxyfluorescein (6-CF; Eastman 0.25 $M$) was prepared in distilled water and the pH was adjusted to 7.4 using sodium hydroxide (Senior and Gregoriadis, 1984). This dye is self-quenching at this concentration and is used as an aqueous marker for liposome leakage. Additionally, 6-CF may be considered as an analogue for industrial payloads. 6-CF solution (25 ml) was added to the lipid and the conical flask was heated to 40°C. The 6-CF-lipid mixture was flushed with nitrogen for 3 minutes, 3 glass beads were added, the flask was stoppered, and mixed with a vortex mixer for 3 minutes to form multilamellar vesicles (MLV's). The suspension of MLV's was sonicated using a bath sonicator (Laboratory Supplies, N.Y.) for 15 minutes. The liposomes were separated from unentrapped material by passing the suspension through a Sepharose CL-6B (Phar-macia) column (1 cm × 20 cm) (Senior and Gregoriadis, 1984).

#### b) Production of lipogel microcapsules

Liposomes prepared as described above were entrapped in calcium alginate-gelatin as follows. The pH of distilled water was adjusted to 12.00 by the dropwise addition of sodium hydroxide (10 $M$). Sodium alginate (1.6% w/v) and gelatin (0.5% w/v) were added and the mixture stirred on a combination hot and stir plate at 40°C until completely dissolved. Following the readjustment of pH to 7.4, the liposomes were stirred into the alginate-gelatin mixture using a stir bar (The temperature of the combined dispersions may be adjusted to account for the thermal tolerance of the liposomes). After the liposomes were added to the alginate-gelatin polymer, the mixture was poured into a Pyrex thin-layer chromatography atomizer (e.g., SMI spray atomizer), and sprayed into a curing bath of calcium chloride (150 ml, 20% w/v) in a 1 liter beaker. Compressed air (at approximately 276 kPa to 414 kPa, 40—60 psi) may be used when encapsulating materials not sensitive to oxidation but when encapsulating polyunsaturated fatty acids or other substances prone to oxidation, compressed nitrogen or argon should be used. The microcapsules were hardened in the calcium chloride for 5 min and collected on a 44 um mesh sieve. The microcapsules were rinsed on the sieve with distilled water and stored in a chloramphenicol solution in distilled water (5 mg/l), under nitrogen at 10°C. The addition of chloramphenicol is necessary to prevent bacterial growth. This step may be omitted if the microcapsules are employed immediately or if an alternative method of sterilisation (e.g., irradiation) is available. The liposomes were encapsulated at up to 20% by weight of the total material. The latency (% of total aqueous solute contained within the liposome) of liposomes was not affected by aerosolisation. Entrapment of the dye was immediately evident in the colour of the Lipogel Microcapsules.

#### c) Production of Aguacultural Lipogel Microcapsules

Liposomes produced according to part (a) above but containing aqueous dietary components such as vitamins are encapsulated in alginate gelatin according to the method of part (b) above.

### Claims

1. A microcapsule for use as an aquacultural feedstuff each microcapsule comprising a payload entrapped in liposomes, the liposomes being encapsulated in a cured hydrocolloid matrix, wherein the payload comprises a nutritional component and the liposomes comprise at least one nutritional lipid.

2. A microcapsule according to claim 1 wherein the matrix is a gelling hydrocolloid selected from agar, alginate, carrageenan, carboxymethyl cellulose, furcellaran, gelatin, pectin, xanthan, dextran, organic polymers and chemically cross-linked albumin, casein and gelatin and mixtures of at least two thereof.

3. A microcapsule according to claim 2 wherein the matrix comprises an alginate salt.

4. A microcapsule according to claim 3 wherein the matrix comprises an alginate salt and gelatin.

5. A microcapsule according to claim 4 wherein the matrix comprises an alginate salt and gelatin in a weight ratio of 10:1 to 1:1.

6. A microcapsule according to claim 5 wherein the matrix comprises an alginate salt and gelatin in a weight ratio of about 3:1.

7. A microcapsule according to any one of claims 2 to 6 wherein the alginate salt is sodium or calcium alginate.

8. A microcapsule according to any one of claims 1 to 7 wherein the matrix is cross-linked by treatment with formaldehyde.

9. A microcapsule according to any one of claim 1 to 7 wherein the matrix is cross-linked by treatment with calcium ions.

10. A microcapsule according to any one of claims 1 to 9 wherein the payload is a protein, mineral carbohydrate, vitamin or amino acid or a mixture of at least two thereof.

11. A microcapsule according to any one of claims 1 to 10 wherein the liposomes comprise at least one unsaturated fatty acid, polyunsaturated fatty acid or sterol.

12. A microcapsule according to claim 11 wherein the liposomes comprise at least one polyunsaturated menhaden phospholipid.

13. A microcapsule according to claim 11 or claim 15 wherein the liposomes comprise cholesterol.

14. A microcapsule according to any one of claims 11 to 13 wherein the liposomes comprise (a) lecithin or dipalmitoyl phosphatidyl choline and (b) cholesterol in a molar ratio of 200:1 to 1:2.

15. A microcapsule according to claim 14 wherein the liposomes comprises (a) lecithin or dipalmitoyl phosphatidyl choline and (b) cholesterol in a molar ratio of 2:1 to 1:2.

16. A microcapsule according to any one of claim 15 wherein the liposomes comprises (a) lecithin or dipalmitoyl phosphatidyl choline and (b) cholesterol in a molar ratio of about 1:1.

17. A microcapsule according to any one of claims 14 to 16 wherein the liposomes comprise soy lecithin.

18. A microcapsule according to any one of claims 1 to 17 wherein the nutritional components of the payload, the nutritional lipid of the liposomes and the hydrocolloid of the matrix form the complete diet of an aquatic organism.

19. A process for producing a microcapsule according to any one of claims 1 to 18 comprising (a) entrapping the payload in liposomes, (b) encapsulating the liposomes in a hydrocolloid matrix and (c) curing the microcapsules.

20. A process according to claim 19 wherein liposomes are admixed with an aqueous solution of the hydrocolloid matrix material and the suspension is nebulised or aerosolised using a suitable carrier gas and sprayed into a curing bath.

21. A formulation comprising microcapsules according to any one of claims 1 to 18 and a carrier or diluent therefor.

22. A method for feeding aquatic livestock comprising providing the livestock with microcapsules according to any one of claims 1 to 18 or a formulation thereof in partial or complete fulfilment of the dietary requirement of the livestock.

23. A method according to claim 22 wherein the aquatic livestock are cultured in a closed system.

**Patentansprüche**

1. Mikrokapsel zur Verwendung als Futtermittel bei der Wasserzucht, wobei die Mikrokapsel eine in Liposomen eingeschlossene, zur Freigabe bestimmte Beschickung enthält und die Liposomen in einer gehärteten hydrokolloiden Matrix eingeschlossen sind und die zur Freigabe bestimmte Beschickung eine Nährsubstanz enthält und die Liposomen wenigstens ein Nährstofflipid enthalten.

2. Mikrokapsel nach Anspruch 1, wobei die Matrix ein gelbildendes Hydrokolloid ist ausgewählt aus Agar, Alginat, Carrageen, Carboxymethylzellulose, Furcellaran, Gelatine, Pektin, Xanthan, Dextran, organischen Polymeren und chemisch vernetztem Albumin, Kasein und Gelatine und Mischungen von mindestens zwei von diesen.

3. Mikrokapsel nach Anspruch 2, wobei die Matrix ein Alginatsalz enthält.

4. Mikrokapsel nach Anspruch 3, wobei die Matrix ein Alginatsalz und Gelatine enthält.

5. Mikrokapsel nach Anspruch 4, wobei die Matrix ein Alginatsalz und Gelatine in einem Gewichtsverhältnis von 10:1 bis 1:1 enthält.

6. Mikrokapsel nach Anspruch 5, wobei die Matrix ein Alginatsalz und Gelatine in einem Gewichtsverhältnis von 3:1 enthält.

7. Mikrokapsel nach einem der Ansprüche 2 bis 6, wobei das Alginatsalz ein Natrium- oder Calziumalginat ist.

8. Mikrokapsel nach einem der vorhergehenden Ansprüche 1 bis 7, wobei die Matrix durch die Behandlung mit Formaldehyd vernetzt ist.

9. Mikrokapsel nach einem der Ansprüche 1 bis 7, wobei die Matrix durch die Behandlung mit Kalziumionen vernetzt ist.

10. Mikrokapsel nach einem der vorhergehenden Ansprüche 1 bis 9, wobei die zur Freigabe bestimmte Beschickung ein Protein, ein mineralischer Kohlenwasserstoff, ein Vitamin oder eine Aminosäure oder eine Mischung von mindestens zwei von diesen ist.

11. Mikrokapsel nach einem der vorhergehenden Ansprüche 1 bis 10, wobei die Liposomen mindestens eine ungesättigte Fettsäure, mehrfach-ungesättigte Fettsäure oder ein Sterin enthalten.

12. Mikrokapsel nach Anspruch 11, wobei die Liposomen wenigstens ein aus dem Menhaden-Fisch (Brevoortia tyrannus) gewonnenes, mehrfach-ungesättigtes Phospholipid enthalten.

13. Mikrokapsel nach Anspruch 11 oder 15, wobei die Liposomen Cholesterin enthalten.

14. Mikrokapsel nach einem der vorhergehenden Ansprüche 11 bis 13, wobei die Liposomen enthalten (a) Lecithin oder Dipalmitoylphosphatidylcholin und (b) Cholesterin in einem molaren Verhältnis von 200:1 bis 1:2.

15. Mikrokapsel nach Anspruch 14, wobei die Liposomen enthalten (a) Lecithin oder Dipalmitoylphosphatidylcholin und (b) Cholesterin in einem molaren Verhältnis von 2:1 bis 1:2.

16. Mikrokapsel nach Anspruch 15, wobei die Liposomen enthalten (a) Lecithin oder Dipalmitoylphosphatidylcholin und (b) Cholesterin in einem molaren Verhältnis von etwa 1:1.

17. Mikrokapsel nach einem der Ansprüche 14 bis 16, wobei die Liposomen Soja-Lecithin enthalten.

18. Mikrokapsel nach einem der Ansprüche 1 bis 17, wobei die Nährstoffbestandteile der zur Freigabe bestimmten Beschickung, das Nährstofflipid der Liposomen und das Hydrokolloid der Matrix die vollständige Nahrung eines in Wasser lebenden Organismus darstellen.

19. Verfahren zur Herstellung einer Mikrokapsel nach einem der Ansprüche 1 bis 18, wobei (a) die zur Freigabe bestimmte Beschickung in Liposomen eingeschlossen wird, (b) die Liposomen in einer hydrokolloiden Matrix eingekapselt werden und (c) die Mikrokapseln gehärtet werden.

20. Verfahren nach Anspruch 19, wobei den Liposomen eine wässrige Lösung eines Materials für die hydrokolloide Matrix zugemischt wird und die Suspension unter Verwendung eines geeigneten Trägergases zu einem Nebel oder Aerosol zerstäubt und in ein Härtungsbad gesprüht wird.

21. Eine Zusammensetzung enthaltend Mikrokapseln nach einem der Ansprüche 1 bis 18 und einen Träger oder ein Verdünnungsmittel hierfür.

22. Ein Verfahren zur Fütterung von sich in Wasser aufhaltenden Lebewesen, bei dem diese mit Mikrokapseln nach einem der Ansprüche 1 bis 18 oder einer diese enthaltenden Zusammensetzung versorgt werden, wobei dem Nahrungsmittelbedarf der Lebewesen teilweise oder vollständig Rechnung getragen wird.

23. Verfahren nach Anspruch 22, wobei die Wasser-Lebewesen in einem geschlossenen System gezüchtet werden.

## Revendications

1. Microcapsule utilisable comme aliment pour l'aquaculture, chaque microcapsule comprenant une charge incluse dans des liposomes, les liposomes étant encapsulés dans une matrice hydrocolloïdale durcie, dans laquelle la charge comprend un composant nutritif et les liposomes comprennent au moins un lipide nutritif.

2. Microcapsule selon la revendication 1, dans laquelle la matrice est un hydrocolloïde gélifiant choisi parmi l'agar-agar, les alginates, le carrageenan, la carboxyméthylcellulose, le furcella- rane, la gélatine, la pectine, le xanthane, le dextrane, les polymères organiques et l'albumine, la caséine et la gélatine réticulées chimiquement, et des mélanges d'au moins deux de ces composés.

3. Microcapsule selon la revendication 2, dans laquelle la matrice comprend un sel alginate.

4. Microcapsule selon la revendication 3, dans laquelle la matrice comprend un sel alginate et de la gélatine.

5. Microcapsule selon la revendication 4, dans laquelle la matrice comprend un sel alginate et de la gélatine dans un rapport en poids de 10:1 à 1:1.

6. Microcapsule selon la revendication 5, dans laquelle la matrice comprend un sel alginate et de la gélatine dans un rapport en poids d'environ 3:1.

7. Microcapsule selon l'une quelconque des revendications 2 à 6, dans laquelle le sel alginate est l'alginate de sodium ou l'alginate de calcium.

8. Microcapsule selon l'une quelconque des revendications 1 à 7, dans laquelle la matrice est réticulée par traitement avec le formaldéhyde.

9. Microcapsule selon l'une quelconque des revendications 1 à 7, dans laquelle la matrice est réticulée par traitement avec des ions calcium.

10. Microcapsule selon l'une quelconque des revendications 1 à 9, dans laquelle la charge est une protéine, un hydrate de carbone minéral, une vitamine ou un acide aminé, ou un mélange d'au moins deux de ces composés.

11. Microcapsule selon l'une quelconque des revendications 1 à 10, dans laquelle les liposomes comprennent au moins un acide gras insaturé, un acide gras polyinsaturé ou un stérol.

12. Microcapsule selon la revendication 11, dans laquelle les liposomes comprennent au moins un phospholipide polyinsaturé de menhaden.

13. Microcapsule selon la revendication 11 ou 15, dans laquelle les liposomes comprennent du cholestérol.

14. Microcapsule selon l'une quelconque des revendications 11 à 13, dans laquelle les liposomes comprennent (a) de la lécithine ou de la dipalmitoyl-phosphatidyl-choline et (b) du cholestérol, dans un rapport molaire de 200:1 à 1:2.

15. Microcapsule selon la revendication 14, dans laquelle les liposomes comprennent (a) de la lécithine ou de la dipalmitoylphosphatidyl-choline et (b) du cholestérol, dans un rapport molaire de 2:1 à 1:2.

16. Microcapsule selon la revendication 15, dans laquelle les liposomes comprennent (a) de la lécithine ou de la dipalmitoylphosphatidyl-choline et (b) du cholestérol, dans un rapport molaire d'environ 1:1.

17. Microcapsule selon l'une quelconque des revendications 14 à 16, dans laquelle les liposomes comprennent la lécithine de soja.

18. Microcapsule selon l'une quelconque des revendications 1 à 17, dans laquelle les composants nutritifs de la charge, le lipide nutritif des liposomes et l'hydrocolloïde de la matrice constituent un régime alimentaire complet d'un organisme aquatique.

19. Procédé de préparation d'une microcapsule selon l'une quelconque des revendications 1 à 18, comprenant (a) l'inclusion de la charge dans les liposomes, (b) l'encapsulation des liposomes dans une matrice hydrocolloïdale, et (c) le durcissement des microcapsules.

20. Procédé selon la revendication 19, dans lequel les liposomes sont mélangés avec une solution aqueuse du matériau de la matrice hydrocolloïdale, et la suspension est nébulisée ou dispersée sous forme d'aérosol, à l'aide d'un gaz vecteur approprié, et pulvérisée dans un bain de durcissement.

21. Formulation comprenant des microcapsules selon l'une quelconque des revendications 1 à 18 et un support ou un diluant pour celles-ci.

22. Procédé pour l'alimentation d'animaux aquatiques, comprenant la fourniture aux animaux de microcapsules selon l'une quelconque des revendications 1 à 18, ou d'une formulation de ces microcapsules, en subvenant partiellement ou totalement aux besoins alimentaires des animaux.

23. Procédé selon la revendication 22, dans lequel les animaux aquatiques sont cultivés dans un système clos.